# EUROPEAN PATENT APPLICATION

(11) **EP 3 741 795 A1**
(43) Date of publication of application: **25.11.2020**
(21) Application number: 18799422.3
(22) Date of filing: 27.01.2018
(51) Int. Cl.: C08J 3/24, C08J 7/12, C08L 29/04, C08K 3/16, A61L 29/04, A61L 29/08, A61L 29/14

(54) **POLYVINYL ALCOHOL COMPOSITION, PREPARATION METHOD THEREFOR AND USE THEREOF**

(30) Priority: 15.01.2018 CN 201810036428
(71) Applicant: SIBIKANG (XIAMEN) NEW MATERIALS CO., LTD., Jimei District Xiamen, Fujian 361023 (CN)
(72) Inventor: CHEN, Shaoyong, Xiamen, Fujian 361023 (CN)
(74) Representative: Dr. Gassner & Partner mbB
(86) International application number: PCT/CN2018/074374
(87) International publication number: WO 2019/136780

(57) **Abstract**

The present invention discloses a polyvinyl alcohol composition, as well as a preparation method and use thereof, wherein raw materials include the following components: 60-90 parts of polyvinyl alcohol; 0.1-3 parts of glutaraldehyde; and the polyvinyl alcohol composition is made by mixing the polyvinyl alcohol in a molten state with the glutaraldehyde in an acidic environment. The present invention has the following advantages and effects: the polyvinyl alcohol composition includes the polyvinyl alcohol, the glutaraldehyde, acid, urea, etc., the glutaraldehyde undergoes cross-linking reaction with the polyvinyl alcohol, and a decomposition temperature of the polyvinyl alcohol composition is increased, while a melting point of the polyethylene alcohol composition is decreased, and the processibility of the polyvinyl alcohol composition is enhanced; a polyvinyl alcohol medical catheter made from the polyvinyl alcohol composition has very good biocompatibility, and is convenient for clinical applications by doctors; furthermore, the polyvinyl alcohol composition can also be used to make various items such as sealing gaskets, degradable flowerpots, and carrying bags, and achieves the effects of the polyvinyl alcohol composition such as low melting temperature, convenience in melting and processing, very good biocompatibility, and broad application range.

## Description

### Technical Field

The present invention relates to the technical field of application of macromolecular polymers, in particular to a polyvinyl alcohol composition as well as a preparation method and use thereof.

### Background Art

Polyvinyl alcohol is a polymer, and its property is between plastics and rubbers. Polyvinyl alcohol has comparatively good bonding property, epithelial flexibility, smoothness, oil resistance, solvent protection resistance, and gas blocking property, etc., and polyvinyl alcohol is sanitary and nontoxic, and is biodegradable under certain conditions. Polyvinyl alcohol is widely used in industries such as textile, food, medicine, construction, wood processing, paper manufacturing, printing, and agriculture, etc. Among them, medical grade polyvinyl alcohol is safe and nontoxic, non-irritating, non-sensitizing, and cytotoxicity-free to the human body, and has comparatively good biocompatibility, and is one of the ideal materials for the manufacturing of medical devices.

The melting of the high polymer is a process in which the crystalline domain transitions from the crystalline state to the liquid state when the crystalline high polymer is heated, and the temperature at this point is the melting point. The macromolecular long-chain of the high polymer exhibits a certain distribution, and the aggregation state thereof is also a typical two-phase structure, i.e., the crystalline phase and the amorphous phase coexist, therefore, the melting of the high polymer is not very sharp, the melting process occurs in a comparatively wide range, and the temperature at which the melting of the high polymer occurs is called the melting point or the melting temperature.

There are lots of hydroxyl groups in the polyvinyl alcohol molecule, and the hydrogen chain formed from them gives the polyvinyl alcohol comparatively good melting temperature, and the melting temperature is close to the decomposition temperature of the polyvinyl alcohol, which lead to the poor processibility. It's difficult to perform thermoplastic processing, and the applicability of the polyvinyl alcohol is restricted.

### Summary of the Invention

The purpose of the invention is to provide a polyvinyl alcohol composition with the effect of low melting temperature.

The above technical purpose of the present invention is achieved by the technical solution below: a polyvinyl alcohol composition, wherein the raw materials of the polyvinyl alcohol composition include the following components in parts by weight: 60-90 parts of polyvinyl alcohol; 0.1-3 parts of glutaraldehyde; and the polyvinyl alcohol composition is made by mixing the polyvinyl alcohol in the molten state with the glutaraldehyde in an acidic environment.

By employing the above technical solution, the polyvinyl alcohol and glutaraldehyde of the raw materials of the polyvinyl alcohol composition undergo cross-linking reaction in the acidic environment to form a three dimensional net structure, which enhance the strength and heat resistance of the polyvinyl alcohol composition. The acidic environment can promote the cross-linking reaction of the glutaraldehyde with the polyvinyl alcohol and increase the decomposition temperature of the polyvinyl alcohol composition, while the melting temperature of the polyvinyl alcohol composition after the cross-linking is decreased, and the processibility of the polyvinyl alcohol composition is increased.

Further, the raw materials of the polyvinyl alcohol composition also include 4-10 parts of urea on the weight basis.

By employing the above technical solution, since the melting point of the high polymer has a direct relationship with the heat enthalpy and the entropy changes during its melting process, the relationship between the melting point and the heat enthalpy and the entropy changes is: Tm=ΔH/ΔS, in the formula, Tm is the melting point of the high polymer, ΔH is the heat enthalpy change of the high polymer during the melting process, and ΔS is the entropy change of the high polymer during the melting process. The factors which can impact ΔH and ΔS can impact the melting point of the high polymer. Among them, the intermolecular force impacts ΔH and the flexibility of the molecular chain impacts ΔS. The stronger the intermolecular force, the stronger the molecular bonding force needed to be overcome when melting, and the higher the melting point of the high polymer. Therefore, by adding urea to the raw materials of the polyvinyl alcohol composition, which is dispersed in the mixture of the raw materials of the polyvinyl alcohol composition, the intermolecular force between the polyvinyl alcohol molecules is reduced, and the melting point of the polyvinyl alcohol composition is substantially decreased.

Further, the raw materials of the polyvinyl alcohol composition also include 5-15 parts of glycerol, 2-10 parts of calcium chloride on the weight basis.

By employing the above technical solution, the raw materials of the polyvinyl alcohol composition include glycerol, which swells and dilutes the polyvinyl alcohol to reduce the intermolecular force between the polyvinyl alcohol molecules, and plays the role of decreasing the melting temperature of the polyvinyl alcohol composition; calcium chloride also plays the role of decreasing the melting temperature of the polyvinyl alcohol composition.

Further, the raw materials of the polyvinyl alcohol composition include the following components in parts by weight: 76-85 parts of polyvinyl alcohol; 5.4-6.5 parts of urea; 8-11 parts of glycerol; 3-5 parts of calcium chloride; 0.4-0.6 parts of glutaraldehyde; 0.1-2 parts of acid; the acid is one of the sulfuric acid aqueous solution having mass fractions of 10-98%, aliphatic carboxylic acid, pyrophosphoric acid, and disulfuric acid.

Further, the polymerization degree of the polyvinyl alcohol is 300-7,000, and the alcoholysis degree is 50-99%.

By employing the above technical solution, the polyvinyl alcohol composition of the present invention has a low melting temperature, in particular, the lower the polymerization degree of the polyvinyl alcohol, the lower the melting temperature; the lower the alcoholysis degree of the polyvinyl alcohol, the lower the melting temperature as well.

Another purpose of the present invention is to provide a preparation method of the polyvinyl alcohol composition, which includes the following steps:
step 1, the polyvinyl alcohol and the acid are mixed and heated at a heating temperature of 140-220°C to melt the polyvinyl alcohol to obtain the acidic polyvinyl alcohol material, followed by pelletization at 140-220°C;
step 2, then a pellet material resulting from the pelletization is uniformly mixed with the glutaraldehyde at 140-220°C to obtain the intermediate material of the polyvinyl alcohol composition;
step 3, the intermediate material of the polyvinyl alcohol composition is soaked in a sodium hydroxide solution having a mass fraction of 5-10 % for 24-48 hours; and
step 4, the intermediate material of the polyvinyl alcohol composition is taken out of the sodium hydroxide solution and washed with deionized water to obtain the polyvinyl alcohol composition.

Further, in step 1, 4-10 parts of urea, 5-15 parts of glycerol, and 2-10 parts of calcium chloride are added to the mixture of the polyvinyl alcohol and the acid, and the mixture is heated at a heating temperature of 140-220°C to melt the polyvinyl alcohol to obtain the acidic polyvinyl alcohol material, followed by pelletization at 140-220°C.

Another purpose of the present invention is to provide a polyvinyl alcohol medical catheter made from the polyvinyl alcohol composition, wherein the material of the polyvinyl alcohol medical catheter is the polyvinyl alcohol composition, and its surface is coated with a coating, and the raw materials of the coating include vinyl pyrrolidone, 2-hydroxyl-4'-(2-hydroxyethoxy)-2-methylpropiophenone, N,N'-methylenebisacrylamide, and ethanol, with a mass ratio of vinyl pyrrolidone,2-hydroxyl-4'-(2-hydroxyethoxy)-2-methylpropiophenone,N,N'-methylenebisacrylamide and ethanol being 3:1:0.04:95.96.

By employing the above technical solution, the surface of the polyvinyl alcohol medical catheter is coated with a coating, upon which forms a layer of lubricating film when contact with water, that increase the surface lubricity of the polyvinyl alcohol medical catheter; during the clinical applications, the lubricating surface of the polyvinyl alcohol medical catheter reduces the stimulation to the cells, doesn't damage the mucous membrane, alleviates the damages to the body cells and tissues, facilitates the medical operation, and relieves the pain of the patients.

Secondly, the surface lubricity of the polyvinyl alcohol medical catheter increases the biocompatibility of the polyvinyl alcohol medical catheter. When the high polymer material without a coating on the surface is exposed to the blood environment, the plasma protein will be rapidly adsorbed onto the surface of the high polymer material, meanwhile the adsorbed plasma protein can induce the adhesion, release, and aggregation of the platelets, which lead to the formation of thrombi, and on the other hand, can readily activate the coagulation factor in the blood and induce the coagulation reaction. To coat a layer of coating on the surface of the polyvinyl alcohol medical catheter can reduce the interaction between the surface of the polyvinyl alcohol medical catheter and the plasma protein and blood cells in the blood, so that it exhibit a good anticoagulation performance.

Further, the preparation method of the polyvinyl alcohol medical catheter comprises the following steps:
step (1), the polyvinyl alcohol, glycerol, calcium chloride, acid, and urea are mixed, extruded with an extruder, and pelletized to obtain the acidic polyvinyl alcohol material, wherein the temperature inside the extruder is 140-220°C;
step (2), the glutaraldehyde and the acidic polyvinyl alcohol material obtained in step (1) are mixed and extruded with an extruder to obtain the polyvinyl alcohol pipes, wherein the temperature inside the extruder is 140-220°C;
step (3), the polyethylene pipes are cooled and soaked in a sodium hydroxide solution having a mass fraction of 5-10% for 24-48 hours;
step (4), the polyvinyl alcohol pipes are taken out of the sodium hydroxide solution, washed with deionized water, and cut into the polyvinyl alcohol catheter;
step (5), the polyvinyl alcohol catheter is coated with a mixed solution and treated with ultraviolet radiation to obtain the polyvinyl alcohol catheter with a coating on the surface, the mixed solution including vinyl pyrrolidone, 2-hydroxyl-4'-(2-hydroxyethoxy)-2-methylpropiophenone, N,N'-methylenebisacrylamide, and ethanol;
step (6), the polyvinyl alcohol catheter with a coating on the surface is placed into the aqueous Artemisia capillaris water-soluble antibacterial solution and soaked therein;
step (7), the polyvinyl alcohol catheter soaked in the aqueous Artemisia capillaris water-soluble antibacterial solution is taken out and sterilized with radiation;
step (8), the polyvinyl alcohol catheter subjected to sterilization with radiation is sealed and packaged to obtain the finished product of polyvinyl alcohol medical catheter.

Another purpose of the present invention is to provide the use of the polyvinyl alcohol composition, wherein the polyvinyl alcohol composition is used to make sealing gaskets, or desiccants, or oil pipes, or carrying bags, or insoles, or packaging bags, or the polyvinyl alcohol composition is mixed with the fertilizer to make the flowerpots.

In view of the above, because the present invention has the following beneficial effects: the polyvinyl alcohol composition includes the polyvinyl alcohol, glutaraldehyde, acid, and urea, etc., and glutaraldehyde undergoes the cross-linking reaction with the polyvinyl alcohol, so the decomposition temperature of the polyvinyl alcohol composition is increased, while the melting point of the polyethylene alcohol composition is decreased, and the processibility of the polyvinyl alcohol composition is enhanced, and it is convenient for melt processing. The polyvinyl alcohol medical catheter made from the polyvinyl alcohol composition has very good biocompatibility, and is convenient for clinical applications by doctors; Furthermore, the polyvinyl alcohol composition can also be used to make various items such as sealing gaskets, degradable flowerpots, and carrying bags, etc., which shows the effects of the polyvinyl alcohol composition such as low melting temperature, good processibility, convenience in melting and processing, very good biocompatibility, and broad application range.

### Brief Description of the Drawings

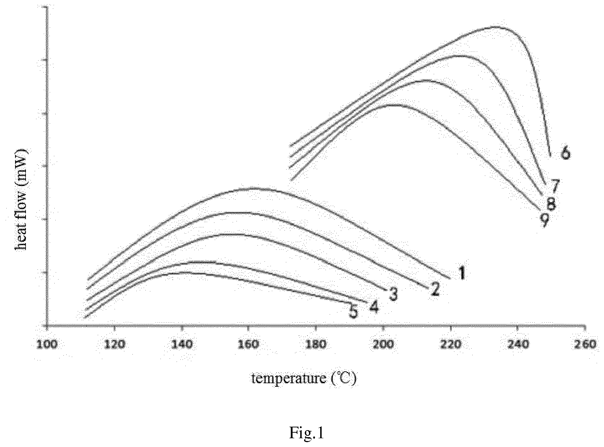

Figure 1 shows DSC test results of examples 1-5 and comparative examples 1-4;
curve 1 is the test result of example 1; curve 2 is the test result of example 2; curve 3 is the test result of example 3, curve 4 is the test result of example 4, curve 5 is the test result of example 5, curve 6 is the test result of comparative example 1, curve 7 is the test result of comparative example 3, curve 8 is the test result of comparative example 4, and curve 9 is the test result of comparative example 2.

### Detailed Description of the Invention

### Example 1

A polyvinyl alcohol composition, with the components of the raw materials thereof being shown in table 1, wherein the acid is selected to be aliphatic carboxylic acid, the polymerization degree of the polyvinyl alcohol is 7,000, and the alcoholysis degree is 99%. The preparation method of the polyvinyl alcohol composition is as follows: step 1, the polyvinyl alcohol, glycerol, calcium chloride, and acid are mixed, extruded with an extruder, and pelletized with a pelletizer to obtain the acidic polyvinyl alcohol material, wherein the extruder is a twin screw extruder, the length diameter ratio is 40-60:1, and the temperature inside the extruder is 160-220°C.

Step 2, the glutaraldehyde and the acidic polyvinyl alcohol material obtained in step 1 are mixed and extruded with an extruder to obtain the intermediate material of the polyvinyl alcohol composition, wherein the temperature inside the extruder is 160-220°C.

Step 3, the intermediate material of the polyvinyl alcohol composition from step 2 is cooled and then soaked in a sodium hydroxide solution having a mass fraction of 5% for 24-48 hours.

Step 4, the intermediate material of the polyvinyl alcohol composition is taken out of the sodium hydroxide solution and washed with deionized water to obtain the polyvinyl alcohol composition.

### Example 2:

A polyvinyl alcohol composition, with the components of the raw materials thereof being shown in table 1, wherein the acid is selected to be pyrophosphoric acid, the polymerization degree of the polyvinyl alcohol is 6,000, and the alcoholysis degree is 80%.

The preparation method of the polyvinyl alcohol composition is as follows: step 1, the polyvinyl alcohol, urea, glycerol, calcium chloride, and acid are mixed, extruded with an extruder, and pelletized with a pelletizer to obtain the acidic polyvinyl alcohol material, wherein the extruder is a twin screw extruder, the length diameter ratio is 40-60:1, and the temperature inside the extruder is 150-180°C.

Step 2, the glutaraldehyde and the acidic polyvinyl alcohol material obtained in step 1 are mixed and extruded with an extruder to obtain the intermediate material of the polyvinyl alcohol composition, wherein the temperature inside the extruder is 150-180°C.

Step 3, the intermediate material of the polyvinyl alcohol composition from step 2 is cooled and then soaked in a sodium hydroxide solution having a mass fraction of 8% for 24-48 hours.

Step 4, the intermediate material of the polyvinyl alcohol composition is taken out of the sodium hydroxide solution and washed with deionized water to obtain the polyvinyl alcohol composition.

### Example 3:

A polyvinyl alcohol composition, with a difference from example 2 in that the components of the raw materials of the polyvinyl alcohol composition are as shown in table 1, wherein the acid is disulfuric acid, the polymerization degree of the polyvinyl alcohol is 4,000, and the alcoholysis degree is 70%.

### Example 4:

A polyvinyl alcohol composition, with the components of the raw materials thereof being shown in table 1, wherein the acid is selected to be the sulfuric acid aqueous solution having a mass fraction of 10%, the polymerization degree of the polyvinyl alcohol is 2,000, and the alcoholysis degree is 50%.

The preparation method of the polyvinyl alcohol composition is as follows:
step 1, the polyvinyl alcohol, urea, glycerol, calcium chloride, and acid are mixed, extruded with an extruder, and pelletized with a pelletizer to obtain the acidic polyvinyl alcohol material, wherein the extruder is a twin screw extruder, the length diameter ratio is 40-60:1, and the temperature inside the extruder is 140-160°C.
Step 2, the glutaraldehyde and the acidic polyvinyl alcohol material obtained in step 1 are mixed and extruded with an extruder to obtain the intermediate material of the polyvinyl alcohol composition, wherein the temperature inside the extruder is 140-160°C.
Step 3, the intermediate material of the polyvinyl alcohol composition from step 2 is cooled and then soaked in a sodium hydroxide solution having a mass fraction of 10% for 24-48 hours.
Step 4, the intermediate material of the polyvinyl alcohol composition is taken out of the sodium hydroxide solution and washed with deionized water to obtain the polyvinyl alcohol composition.

### Example 5:

A polyvinyl alcohol composition, with a difference from example 4 in that the components of the raw materials of the polyvinyl alcohol are as shown in table 1, wherein the acid is the sulfuric acid aqueous solution having a mass fraction of 98%. The polymerization degree of the polyethylene is 300, the alcoholysis degree is 50%, in the preparation method of the polyethylene composition, the temperature inside the extruder in step 1 is 140-220°C, and the temperature inside the extruder in step 2 is 140-220°C.

### Example 6:

a polyvinyl alcohol medical catheter, wherein the material thereof is the polyvinyl alcohol composition, and the components of the raw materials of the polyvinyl alcohol composition are as shown in table 1, wherein the acid is the sulfuric acid aqueous solution having a mass fraction of 60%, the polymerization degree of the polyvinyl alcohol is 300-7,000, and the degree of alcoholysis is 50-99%. The material of the polyvinyl alcohol medical catheter is the polyvinyl alcohol composition, and it's surface is coated with a coating, and the raw materials of the coating included vinyl pyrrolidone, 2-hydroxyl-4'-(2-hydroxyethoxy)-2-methylpropiophenone, N,N'-methylenebisacrylamide, and ethanol.

The preparation method of the polyvinyl alcohol medical catheter is as follows:
step (1), the polyvinyl alcohol, glycerol, calcium chloride, acid, and urea are mixed, extruded with an extruder, and pelletized to obtain the acidic polyvinyl alcohol material, wherein the extruder is a twin screw extruder, the length diameter ratio of the screw is 40-60:1, and the temperature inside the extruder is 140-220°C.
Step (2), the glutaraldehyde and the acidic polyvinyl alcohol material obtained in step (1) are mixed and extruded with an extruder to obtain the polyvinyl alcohol pipes, wherein the temperature inside the extruder is 140-220°C.
Step (3), the polyethylene pipes are cooled and then soaked in a sodium hydroxide solution having a mass fraction of 5-10% for 24-48 hours.
Step (4), the polyvinyl alcohol pipes are taken out of the sodium hydroxide solution, washed with deionized water, and cut into the polyvinyl alcohol catheter.
Step (5), the polyvinyl alcohol catheter is coated with a mixed solution and treated with ultraviolet radiation to obtain the polyvinyl alcohol catheter with a coating on the surface, and the mixed solution include vinyl pyrrolidone, 2-hydroxyl-4'-(2-hydroxyethoxy)-2-methylpropiophenone, N,N'-methylenebisacrylamide, and ethanol. Among them, the mass ratio of the components of the mixed solution was: vinyl pyrrolidone:2-hydroxyl-4'-(2-hydroxyethoxy)-2-methylpropiophenone:N,N'-methylenebisacrylamide:ethanol = 3:1:0.04:95.96. The range of the ultraviolet wavelength during the treatment with ultraviolet radiation is 365-370 nm.
Step (6), the polyvinyl alcohol catheter with a coating on the surface is placed into the aqueous Artemisia capillaris water-soluble antibacterial solution and then soaked therein.
Step (7), the polyvinyl alcohol catheter soaked in the aqueous Artemisia capillaris water-soluble antibacterial solution is taken out and sterilized with radiation, wherein cobalt 60 is used for the sterilization with radiation, and the radiant quantity is 0.5-1.5KGy.

Among that, the preparation method of each 100 ml of the aqueous Artemisia capillaris water-soluble antibacterial solution is as follows:
a, 10 g of the material is weighed and added into an Erlenmeyer flask, wherein the material is a whole plant of the Artemisia capillaris;
b, water is added: 150 g of distilled water is added into the Erlenmeyer flask;
c, leaching: the Erlenmeyer flask is thermostatically treated at 60°C for 80 minutes;
d, volume metering: the mixture in the Erlenmeyer flask is poured into a volumetric flask through a funnel, then water is added into the volumetric flask to dilute to 500 mL, and the mixture is shaked multiple times and let stand for 10 minutes;
e, filtration: the mixture is filtered with a funnel to obtain 100 mL of filtrate, i.e., the aqueous Artemisia capillaris water-soluble antibacterial solution.

Step (8), the polyvinyl alcohol catheter subjected to sterilization with radiation is sealed and packaged to obtain the finished product of the polyvinyl alcohol medical catheter.

### Example 7:

A sealing gasket made from any of the polyvinyl alcohol composition of examples 1-5. A plastic injection molding machine is employed to inject the polyvinyl alcohol composition into the mould to be shaped therein to obtain the sealing gasket. Since the polyvinyl alcohol has the water absorbency, the sealing gasket swells after absorption of water, which increases the sealing property of the sealing gasket.

### Example 8:

A desiccant made from any of the polyvinyl alcohol composition of examples 1-5. The preparation method is as follows: the polyvinyl alcohol composition is cut into lumps to obtain the desiccant. The polyvinyl alcohol in the desiccant has water absorbency, and it absorbs water and has a good drying effect.

### Example 9:

An oil pipe made from any of the polyvinyl alcohol composition of examples 1-5. The preparation method of the oil pipe is as follows: step 1), the polyvinyl alcohol, glycerol, calcium chloride, acid, and urea are mixed, extruded with an extruder, and pelletized to obtain the acidic polyvinyl alcohol material, wherein the extruder is a twin screw extruder, the length diameter ratio of the screw is 40-60:1, and the temperature inside the extruder is 140-220°C.

Step 2), the glutaraldehyde and the acidic polyvinyl alcohol material obtained in step 1) are mixed and extruded with an extruder to obtain the polyvinyl alcohol pipes, wherein the temperature inside the extruder is 140-220°C.

Step 3), the polyethylene pipes are cooled and then soaked in a sodium hydroxide solution having a mass fraction of 5-10% for 24-48 hours.

Step 4), the polyvinyl alcohol pipes are taken out of the sodium hydroxide solution, washed with deionized water, and cut into the oil pipe. The oil pipe obtained has the advantages of good air barrier property and smoothness of the inner wall of the oil pipe.

### Example 10:

A carrying bag made from any of the polyvinyl alcohol composition of examples 1-5, wherein the carrying bag is made by employing a blow molding process and is susceptible to degradation in the environment after usage.

### Example 11:

An insole made from any of the polyvinyl alcohol composition of examples 1-5, wherein during the manufacturing a plastic injection molding machine is employed to inject the polyvinyl alcohol composition into the mould to be shaped therein to obtain the insole. The raw materials of the insole contains the polyvinyl alcohol, and the polyvinyl alcohol has the water absorbency, which enable the insole to absorb the sweat.

### Example 12:

A flowerpot made by mixing any of the polyvinyl alcohol composition of examples 1 -5 with the fertilizer. During the manufacturing the polyvinyl alcohol composition is heated to 140-220°C, and after the fertilizer is added and stirred until homogeneous, the mixture is poured into the mould to be cooled and shaped to obtain the flowerpot. During the usage of the flowerpot, the polyvinyl alcohol continuously degraded, and the fertilizer in the flowerpot is continuously released to provide the nutrients for the plant in the flowerpot.

### Example 13:

A packaging bag made from any of the polyvinyl alcohol composition of examples 1-5, wherein the packaging bag is made by employing a blow molding process and is susceptible to degradation in the environment after usage.

Comparative example 1: a polyvinyl alcohol with a polymerization degree of 300-7,000 and an alcoholysis degree of 50-99%.

Comparative example 2: a polyvinyl alcohol composition with the components of the raw materials thereof are as shown in table 1, wherein the polymerization degree of the polyvinyl alcohol is 300-7,000, and the alcoholysis degree is 50-99%.

The preparation method of the polyvinyl alcohol composition is as follows: step A, the polyvinyl alcohol, glycerol, and calcium chloride are mixed, extruded with an extruder, and pelletized with a pelletizer to obtain the polyvinyl alcohol material, wherein the extruder is a twin screw extruder, the length diameter ratio is 40-60:1, and the temperature inside the extruder is 140-220°C.

Step B, the glutaraldehyde and the polyvinyl alcohol material obtained in step A are mixed and extruded with an extruder to obtain the polyvinyl alcohol composition, wherein the temperature inside the extruder was 140-220°C.

Comparative example 3: a polyvinyl alcohol composition with the components of the raw materials thereof are as shown in table 1, wherein the polymerization degree of the polyvinyl alcohol is 300-7,000, and the alcoholysis degree is 50-99%.

The preparation method of the polyvinyl alcohol composition is as follows: the polyvinyl alcohol, glycerol, and calcium chloride are mixed, extruded with an extruder, and pelletized with a pelletizer to obtain the acidic polyvinyl alcohol material, wherein the extruder is a twin screw extruder, the length diameter ratio is 40-60:1, and the temperature inside the extruder is 140-220°C, and it is pelletized to obtain the polyvinyl alcohol composition. Comparative example 4: a polyvinyl alcohol composition with the components of the raw materials thereof are as shown in table 1, wherein the polymerization degree of the polyvinyl alcohol is 300-7,000, and the alcoholysis degree is 50-99%.

The preparation method of the polyvinyl alcohol composition is as follows: the polyvinyl alcohol, urea, glycerol, and calcium chloride are mixed, extruded with an extruder, and pelletized with a pelletizer to obtain the acidic polyvinyl alcohol composition, wherein the extruder is a twin screw extruder, the length diameter ratio is 40-60:1, and the temperature inside the extruder is 140-220°C, and it is pelletized to obtain the polyvinyl alcohol composition.

**Table 1**

| | Polyvinyl alcohol (Kg) | Urea (Kg) | Glycerol (Kg) | Calcium chloride (Kg) | Glutaraldehyde (Kg) | Acid (Kg) |
|---|---|---|---|---|---|---|
| Example 1 | 76 | 0 | 8 | 3 | 0.4 | 0.1 |
| Example 2 | 76 | 5.4 | 8 | 3 | 0.4 | 0.1 |
| Example 3 | 85 | 6.5 | 11 | 5 | 0.6 | 0.6 |
| Example 4 | 60 | 10 | 5 | 10 | 0.1 | 1 |
| Example 5 | 90 | 4 | 15 | 2 | 3 | 2 |
| Example 6 | 85 | 6.5 | 11 | 5 | 0.6 | 0.6 |
| Comparative Example 2 | 76 | 0 | 8 | 3 | 0.4 | 0 |
| Comparative Example 3 | 76 | 0 | 8 | 3 | 0 | 0 |
| Comparative Example 4 | 76 | 5.4 | 8 | 3 | 0 | 0 |

The melting temperature test of the polyvinyl alcohol composition:
The polyvinyl alcohol composition of examples 1-5 and comparative examples 1-4 and the polyvinyl alcohol of comparative example 1 are taken and subjected to the DSC test respectively, wherein DSC is Differential Scanning Calorimetry. The test results are shown in figure 1, wherein curve 1 is the test result of example 1, curve 2 is the test result of example 2, curve 3 is the test result of example 3, curve 4 is the test result of example 4, curve 5 is the test result of example 5, curve 6 is the test result of comparative example 1, curve 7 is the test result of comparative example 3, curve 8 is the test result of comparative example 4, and curve 9 is the test result of comparative example 2. The results of figure 1 show that all the melting points of the polyvinyl alcohol composition of examples 1-6 are apparently lower than the melting points of the polyvinyl alcohol of comparative example 1 and the polyvinyl alcohol composition of comparative examples 2-4.

## Claims

1. A polyvinyl alcohol composition, wherein raw materials of the polyvinyl alcohol composition include the following components in parts by weight: 60-90 parts of polyvinyl alcohol; 0.1-3 parts of glutaraldehyde; and the polyvinyl alcohol composition is made by mixing the polyvinyl alcohol in a molten state with the glutaraldehyde in an acidic environment.

2. The polyvinyl alcohol composition according to claim 1, wherein the raw materials of the polyvinyl alcohol composition further include 4-10 parts of urea on the weight basis.

3. The polyvinyl alcohol composition according to claim 2, wherein, the raw materials of the polyvinyl alcohol composition further include 5-15 parts of glycerol and 2-10 parts of calcium chloride on the weight basis.

4. The polyvinyl alcohol composition according to claim 3, wherein the raw materials of the polyvinyl alcohol composition include the following components in parts by weight: 76-85 parts of polyvinyl alcohol; 5.4-6.5 parts of urea; 8-11 parts of glycerol; 3-5 parts of calcium chloride; 0.4-0.6 part of glutaraldehyde; 0.1-2 parts of acid; and the acid is one of a sulfuric acid aqueous solution having mass fractions of 10-98%, aliphatic carboxylic acid, pyrophosphoric acid, and disulfuric acid.

5. The polyvinyl alcohol composition according to claim 1, wherein a polymerization degree of the polyvinyl alcohol is 300-7,000, and an alcoholysis degree of the polyvinyl alcohol is 50-99%.

6. A preparation method of the polyvinyl alcohol composition according to claim 1, comprising:
step 1: mixing polyvinyl alcohol with acid and heating at a heating temperature of 140-220°C to melt the polyvinyl alcohol, to obtain an acidic polyvinyl alcohol material, and then pelletizing at 140-220°C;
step 2: then uniformly mixing a pellet material resulting from pelletization with the glutaraldehyde at 140-220°C to obtain an intermediate material of the polyvinyl alcohol composition;
step 3: soaking the intermediate material of the polyvinyl alcohol composition in a sodium hydroxide solution having a mass fraction of 5-10 % for 24-48 hours; and
step 4: taking the intermediate material of the polyvinyl alcohol composition out of the sodium hydroxide solution and washing with deionized water to obtain the polyvinyl alcohol composition.

7. The preparation method of the polyvinyl alcohol composition according to claim 6, wherein in step 1, 4-10 parts of urea, 5-15 parts of glycerol, and 2-10 parts of calcium chloride are added to a mixture of the polyvinyl alcohol and the acid, and heating at a heating temperature of 140-220°C to melt the polyvinyl alcohol to obtain the acidic polyvinyl alcohol material, followed by pelletization at 140-220°C.

8. A polyvinyl alcohol medical catheter, wherein the polyvinyl alcohol medical catheter is made from the polyvinyl alcohol composition of claim 4, and a surface of the polyvinyl alcohol medical catheter is coated with a coating, and raw materials of the coating include vinyl pyrrolidone, 2-hydroxyl-4'-(2-hydroxyethoxy)-2-methylpropiophenone, N,N'-methylenebisacrylamide, and ethanol, with a mass ratio of vinyl pyrrolidone,2-hydroxyl-4'-(2-hydroxyethoxy)-2-methylpropiophenone,N,N'-methylenebisacrylamide and ethanol being 3:1:0.04:95.96.

9. The polyvinyl alcohol medical catheter according to claim 8, wherein a preparation method of the polyvinyl alcohol medical catheter comprises the following steps:
Step (1): mixing polyvinyl alcohol, glycerol, calcium chloride, acid and urea, extruding with an extruder, and pelletizing to obtain an acidic polyvinyl alcohol material, wherein a temperature inside the extruder is 140-220°C;
step (2): mixing glutaraldehyde and the acidic polyvinyl alcohol material obtained in step (1) and extruding with the extruder to obtain polyvinyl alcohol pipes, wherein the temperature inside the extruder is 140-220°C;
step (3): cooling and then soaking the polyethylene pipes in a sodium hydroxide solution having a mass fraction of 5-10% for 24-48 hours;
step (4): taking the polyvinyl alcohol pipes out of the sodium hydroxide solution, washing with deionized water, and cutting into polyvinyl alcohol catheters;
step (5): coating the polyvinyl alcohol catheters with a mixed solution and treating with ultraviolet radiation to obtain polyvinyl alcohol catheters with coatings on surfaces, wherein the mixed solution includes vinyl pyrrolidone, 2-hydroxyl-4'-(2-hydroxyethoxy)-2-methylpropiophenone, N,N'-methylenebisacrylamide, and ethanol;
step (6): placing the polyvinyl alcohol catheters with the coatings on the surfaces into an aqueous Artemisia capillaris water-soluble antibacterial solution and soaking therein;
step (7): taking out the polyvinyl alcohol catheters soaked in the aqueous Artemisia capillaris water-soluble antibacterial solution and sterilizing with radiation; and
step (8): sealing and packaging the polyvinyl alcohol catheters subjected to sterilization with radiation to obtain finished products of the polyvinyl alcohol medical catheters.

10. Use of the polyvinyl alcohol composition according to claim 4, wherein the polyvinyl alcohol composition is used to make sealing gaskets, desiccants, oil pipes, carrying bags, insoles, or packaging bags, or the polyvinyl alcohol composition is mixed with a fertilizer to make flowerpots.
